(19) **Europäisches Patentamt**
**European Patent Office**
**Office européen des brevets**

(11) **EP 2 004 822 B1**

(12) **EUROPEAN PATENT SPECIFICATION**

(45) Date of publication and mention
of the grant of the patent:
**12.06.2013 Bulletin 2013/24**

(51) Int Cl.:
*C12N 15/10* (2006.01)

(21) Application number: **06820725.7**

(22) Date of filing: **31.07.2006**

(86) International application number:
**PCT/IB2006/002079**

(87) International publication number:
**WO 2007/113614 (11.10.2007 Gazette 2007/41)**

(54) **A METHOD FOR RAPID ISOLATION OF RNA AND A KIT THEREOF**

SCHNELLES RNA-ISOLIERUNGSVERFAHREN UND KIT DAFÜR

PROCEDE D'ISOLEMENT RAPIDE DE L'ARN ET KIT POUR LEDIT ISOLEMENT

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.03.2006 IN DE08852006**

(43) Date of publication of application:
**24.12.2008 Bulletin 2008/52**

(73) Proprietor: **Council of Scientific and Industrial Research**
**New Delhi 110 011 (IN)**

(72) Inventors:
• **GHWANA, Sanjay**
**Palampur (IN)**
• **SINGH, Kashmir**
**Palampur (IN)**
• **RAIZADA, Jyoti**
**Palampur (IN)**
• **RANI, Arti**
**Palampur (IN)**
• **BHARDWAJ, Pradeep, Kumar**
**Palampur (IN)**

• **KUMAR, Sanjay**
**Palampur (IN)**

(74) Representative: **Fiussello, Francesco et al**
**Studio Torta S.p.A.**
**Via Viotti, 9**
**10121 Torino (IT)**

(56) References cited:
**WO-A-98/45311      WO-A1-02/057289**
**US-A- 5 973 137**

• BUGOS R C ET AL: "RNA ISOLATION FROM PLANT TISSUES RECALCITRANT TO EXTRACTION IN GUANIDINE" BIOTECHNIQUES, INFORMA LIFE SCIENCES PUBLISHING, WESTBOROUGH, MA, US, vol. 19, no. 5, November 1995 (1995-11), pages 734-737, XP001055648 ISSN: 0736-6205 cited in the application
• MASON MICHAEL G ET AL: "Research note: Rapid isolation of total RNA and genomic DNA from Hakea actities" FUNCTIONAL PLANT BIOLOGY, vol. 29, no. 8, 2002, pages 1013-1016, XP009078444 ISSN: 1445-4408

**EP 2 004 822 B1**

## Description

### Field of invention:

The present invention relates to a method for rapid isolation of RNA.

More particularly, it relates to a method for isolation of RNA using two-solution system. The present invention also relates to a RNA isolation kit.

### Background and prior art of invention:

Extensive research has been undertaken in the field of molecular biology to facilitate the deciphering of underlying mechanisms of gene expression, signal transduction, gene regulation and transcriptome analysis. This involves a whole gamut of techniques such as reverse transcription polymerase chain reaction (hereinafter, referred to as RT-PCR), northern hybridization, construction of cDNA libraries and *in vitro* translation. Substantially pure and undegraded RNA is a fundamental requisite for all the above-mentioned techniques.

Several compositions and procedures for isolation of RNA have been described as mentioned below:

Current Protocols in Molecular Biology (Ausubel, F.M., Brent, R., Kingston, R. E., Moore, D.D., Seidman, J.G., Smith, J.A. and Struhl, K. Eds,1994. John Wiley and Sons, Inc. USA) described a RNA isolation protocol, which involves cell lysis and protein removal by phenol/SDS, followed by selective precipitation of RNA using lithium chloride. In brief, the tissue is ground in liquid nitrogen using mortar pestle followed by transfer to grinding buffer (0.18 M Tris, 0.09 M LiCl, 4.5 mM EDTA and 1% SDS, pH 8.2) containing phenol saturated with 0.2M Tris , 0.1 M LiCl and 5 mM EDTA solution (pH 8.2) in a ratio of 3:1. After homogenization in a polytron, proteins are removed from the aqueous phase by several re-extractions with saturated phenol and chloroform. The aqueous phase so obtained is precipitated several times with 8 M LiCl. Because of repeated precipitation steps, the procedure becomes time consuming, tiring and expensive.

Cox, R.A. (in Methods in Enzymology 1968, Grosmann, L. and Moldave, K. Eds. Vo1.12 B, pp. 120-129, Academic Press, Orlando, FL) described RNA isolation method using guanidine hydrochloride. Guanidine hydrochloride is a strong inhibitor of ribonucleases. However, the procedure is time-consuming. Guanidine salts employed for the isolation of RNA are extremely poisonous. Moreover, several plant tissues are recalcitrant to extraction in guanidinium salts. Negligible or no RNA is obtained from certain tissues by extraction in guanidine [R.C. Bugos, V.L. Chiang, X-H. Zhang, E.R. Campbell, G.K. Podila, W.H. Campbell. RNA isolation from plant tissues recalcitrant to extraction in guanidine, Biotechniques 19 (1995) 734-737].

Chirgwin, J.M., Przybyla, A.E., Macdonald, R.J.,& Rutter, W.J. (1979) Biochemistry 18: 5294-5299, described a method for isolation of RNA using strong denaturant guanidine thiocyanate, in which both cation and anion are potent chaotropic agents. In this method, the tissue is homogenized in a solution containing guanidine thiocyanate (4M), sodium N-laurylsarcosine (0.5%), sodium citrate, pH 7.0 (25mM) and beta-mercaptoethanol (0.1M). The supernatant is acidified with 1M acetic acid, and RNA is precipitated with 0.75 volume of absolute alcohol at -20°C. Further steps involve dissolution of RNA pellet obtained after centrifugation *in* guanidine chloride solution (7.5M), buffered with sodium citrate pH 7.0 containing 5mM dithiothreitol. Re-precipitation is done in acetic acid and ethanol for 3-4h at -20°C. Another dissolution and re-precipitation step is involved for isolation of RNA. A modification of this procedure involves separating RNA from the homogenate by ultracentrifugation through a cesium chloride gradient. This method also uses toxic gunanidine salt and is very time consuming.

Wallace, D.M. (in Methods in Enzymology, 152:33-41; 1987) described phenol-based extractions of RNA from biological tissues. Using warm buffer-saturated phenol, aqueous phase obtained was re-extracted with chloroform-isoamyl alcohol and buffer-saturated phenol. Re-extraction is repeated and RNA is precipitated with ethanol by incubating overnight. The procedure is time consuming as it requires overnight precipitation procedures. Further, there is no protection to RNA from RNases in the aqueous phase.

US Patent 4,843,155 and Chomczynski, P. and Sacchi, N. (1987) Anal. Biochem. 162:156-159 described a procedure for simultaneous isolation of RNA, DNA and protein. This is also termed as acid guanidinium-phenol-chloroform (hereinafter called as, AGPC) method. In this procedure, tissue is homogenized in a solvent consisting of 4M guanidinium thiocyanate, 25mM sodium citrate, pH 7; 0.5% sarkosyl, 0.1M 2-mercaptoethanol. Phase separation is done by addition of phenol saturated with water and 0.2M sodium acetate, pH 4.0, and chloroform-isoamyl alcohol mixture (49:1) by vigorous mixing and centrifugation. RNA compartmentalizes to the aqueous phase whereas DNA and proteins are present in the organic and the interphase. Precipitation is done by the addition of equal volume of isopropanol, the resulting RNA pellet is dissolved in homogenization solution and precipitated with 1 volume of isopropanol at -20°C for 1 hour. Washing is done in 70% ethanol and RNA pellet is dissolved in 0.5% SDS. This procedure requires at least 4 hours for RNA isolation.

Method developed by Chomczynski, P. and Sacchi, N. (1987) Anal. Biochem. 162:156-159 involving modification of guanidinium thioicyanate method of Chirgwin, J.M., Przybyla, A.E., Macdonald, R.J.,& Rutter, W.J.(1979) Biochemistry 18: 5294-5299, using guanidine thiocyanate-phenol-cholroform at acidic pH is rapid but this AGPC method usually yields very high amounts of genomic DNA contamination.

Siebert, P.D. and Chenchik, A. (1993) Nucl. Acid Res. 21(8): 2019-2020 made simple modifications of AGPC method by addition of a selective RNA precipitation step. This involved addition of one-third volume of 95% ethanol following lysis in guanidine thiocyanate. Isopropanol precipitation step was also reduced from 1h to 30 min. DNA contamination was significantly reduced. But still the method required 3-4h for RNA isolation and is not suitable for tissues resistant to guanidine salts.

United States Patent 5,945,515 by Chomczynski, P. (1999) disclosed a solution for simultaneous isolation of RNA, DNA and proteins. The solution consisted of guanidinium thioicyanate in 40-60% phenol, glycerol as phenol solubilizer and a buffer to maintain the solvent pH at or about 4. The mixture is a homogenous mixture (monophasic). Phase separation is effected by the addition of chloroform at 10% that results in partitioning of RNA in the aqueous phase. Proteins and DNA are concentrated in the organic or the interphase. RNA precipitation is carried out by the addition of equal volume of isopropanol to the aqueous phase. The RNA pellet is obtained by centrifugation that is washed with 70% ethanol and allowed to dry. The presence of very high concentration (2-5M) of guanidinium salts makes the solution extremely hazardous to health. Further, tissues recalcitrant to guanidinium salts do not yield any RNA.

US Patent 5,777,099 by Mehra, M. (1998) describes a method for separation of RNA from liquid samples of biological origin, by contacting with a biphasic solution wherein the upper phase is phenol and the lower phase is aqueous phase containing guanidinium salt, a buffer, and urea. Separation of aqueous phase is effected by addition of water-insoluble solvent such as chloroform. RNA is recovered from the resulting aqueous phase. The presence of guanidinium salts makes the solution extremely hazardous to health. Further, tissues recalcitrant to guanidinium salts do not yield any RNA.

U.S. Pat. No. 5,010,183, by Macfarlane, D. E. (1991) describes the ability of cationic surfactants to lyse cells and simultaneously precipitate RNA and DNA from solution. This method differs fundamentally from those described above in that its first step renders the RNA insoluble, whereas in the above described methods the first step is to solubilize RNA. In this method, a 2% solution of the surfactant benzyldimethyl-n-hexadecylammonium chloride together with 40% urea and other additives are added to a cell suspension, and the mixture is centrifuged. The pellet is resuspended in ethanol, from which the RNA and DNA is precipitated by the addition of a salt. In attempts to apply this method to blood, the inventor himself found that the use of the latter surfactant and other commercially available surfactants results in inefficient precipitation of RNA and incomplete lysis of blood cells.

US patent No. 5,300,635 by Macfarlane, D. E. (1994) discloses a novel method for isolating nucleic acids from biological samples, most particularly blood, using selected quaternary amine surfactants. The selected quaternary amine is produced through the reaction of a quaternary amine hydroxide and an acid of the group consisting of phosphoric, sulfuric, formic, acetic, propionic, oxalic, malonic, succinic and citric. The quaternary amine is either an acyltrimethylanamonium or an acylbenzyldimethylammonium, where the acyl group contains 12, 14, 16 or 18 carbons. This method employs 4M guanidinium isothiocyanate solution for dissociation of nucleic acids from nucleic acid/ quaternary amine complex which is quite hazardous. Further, the method is mostly suitable for isolation of RNA from blood and animal tissue, whereas no experiment has been conducted on plant tissue.

Feramisco, J.R., Helfman, D.M., Smart, J.E., Burridge, K., and Thomas, G.P. (in Molecular Cloning (Maniatis, T., Fritsch, E. F., and Sambrook, J., Eds. (1982), PP. 194-195, Cold Spring Harbor Laboratory, Cold Spring Harbor, NY) reports another RNA isolation procedure wherein RNA-containing samples are homogenized in a solution of 4M guanidinium thiocyanate, 20% sodium lauryl sarcosine, and 2-mercaptoethanol. Following homogenization, an equal volume of heated phenol (60°C) and sodium acetate pH 5.2 are added to the homogenate. Next, an equal amount of chloroform is added, and the mixture is cooled and centrifuged. The aqueous phase is decanted and re-extracted several times with a phenol/chloroform solution in order to maximize the yield. The procedure requires a skilled technician and takes four to five hours to complete and also employs guanidinium thiocyanate which is quite hazardous.

United States Patent No. 20040019196 by Bair, R J Jr., Heath, EM., Meehan, H, Paulsen, K E, Wages, J M. Jr.(2004) describes a method for isolation of RNA from blood, mammalian cells, plant tissue, bacteria and fungi. Initially, 200 μL of Lysing/Binding Solution (4 LiCl, 5% Triton X-100, 5% DGME (diethylene glycol monoethyl ether), 10 mM EDTA, 10 mM TCEP (Tris (carboxyethyl) phosphine), 1% sodium tungstate, in 100 mM TRIZMA at pH 8.8) was added for each 30 mg of tissue sample in a tube. Tissue was homogenized *in* a roto-stator at low speed and then the speed was increased to complete homogenization for an additional minute. After homogenization, 200 μl of homogenized lysate was added to a pre-clear column (Gentra). Depending upon the tissue and extent of homogenization, the pre-clear column trapped particulates, while allowing the bulk of the applied lysis volume containing the RNA to wash through the filter. After centrifugation in the pre-clear column, the cleared lysate at the bottom of the pre-clear tube was vortexed briefly. Then, the entire volume of cleared lysate (about.200 μL) was pipetted onto the purification column, which contained a borosilicate glass fiber membrane (Whatman D glass fiber membrane) within a basket and placed inside a 2 ml microfuge tube. The microfuge tube was then spun at maximum speed in a microcentrifuge for 1 minute. The microfuge tube was then turned 180 degree in the microcentrifuge and centrifuged for an additional 2 minutes. After centrifugation of lysate and subsequent binding of RNA to the borosilicate membrane surface, 200 µL of Wash I Solution (5 M LiCl and 55% ethanol) was added to the column material and spun at maximum speed in a microcentrifuge for 1 minute. The basket containing the membrane was then transferred to a new microfuge tube and 200 µof Wash II Solution (5 mM EDTA, 70% ethanol, in 100 mM Tris HCl at pH 7.6) was added to the column material and spun at maximum speed in a microcentrifuge for 1 minute. The Wash II Solution addition and centrifugation steps were repeated once. To elute the RNA from the solid support, the basket containing the membrane was transferred to a new microfuge tube and 50 µL of DEPC-treated water was added to the column material and spun at maximum speed for 1 minute. The patent doesn't not provide any experiment /data on isolation of RNA from plant tissues which are usually very difficult due to presence of polysaccharides and other contaminating material. The use of lithium chloride and other accessories renders this method expensive.

United States Patent No. 5,973,137 by Heath, E M. (1999) describes a method for isolation of RNA from human whole blood, plant and animal tissues, cultured cells, body fluids, yeast, and bacteria. Inventor has used a "Cell Lysis Reagent," which includes an anionic detergent (salts e.g., sodium, potassium, and lithium salts of dodecyl sulfate as well as N-lauroyl sarcosine; 1.8-2.2% weight/volume) dissolved in water buffered with sodium citrate and citric acid at concentration of 66-70 mM and 130-134 mM, concentration, respectively. In addition to the anionic detergent and buffer, the reagent includes a chelating agent such as ethylene diamine tetraacetate (EDTA) as a preferred reagent in an amount effective to reduce DNase activity. The second reagent, referred to therein as a "Protein-DNA Precipitation Reagent," includes a sodium or potassium salt such as sodium chloride, sodium acetate, potassium chloride and potassium acetate in a relatively high salt concentration (3.8-4.2 M) dissolved in water. Inventor has used anionic detergent such as sodium dodecyl sulfate as inhibitor of RNAses. RNAses is a major problem in the tissues, particularly in old and stressed tissues. Anionic detergents are mild inhibitors of RNAses (Wallace, D.M., in Methods in Enzymology, 152: 33-41; 1987; please refer to the 7th line from the top on page 37) and hence would not be suitable for the tissues with high RNAses. Secondly, a very high salt concentration (3.8 to 4.2 M) used in the method would cause non-specific RNA aggregation leading to loss of, particularly, the minor RNA species (Wallace, D.M., in Methods in Enzymology, 152: 33-41; 1987; please refer to the 2nd last para from the bottom on page 36).

The drawbacks in the prior art are:

The methods use guanidinium salts (Guanidine hydrochloride and guanidine thiocyanate) as a major constituent. Guanidine hydrochloride is extremely hazardous and rated as harmful by CHIP (UK Chemicals Hazard information and Packaging) and toxic by HCS (US Hazard Communication standards). Oral Rat LD50 = 475 mg/kg.

Tissues recalcitrant to guanidine salts do not yield RNA or the yield is too poor to allow any further use.

Available reagents in the market are very expensive.

Non-guanidine hydrochloride based procedures are very lengthy.

Solutions/protocols not involving the use of guanidine salts are not suitable for tissues with high RNAses and also would lead to loss to RNA species due to the high salt concentration used to inhibit RNase activity.

Some of the methods use cesium chloride for isolation of RNA on ultracentrifuge, which is a very expensive instrument. Also, skilled personnel are required to operate the ultracentrifuge taking all safety precautions, thus, the procedure becomes very expensive and time consuming.

WO 98/45311 by Life Technologies Inc (1998) discloses the isolation of plant RNA where a phenol, a phenol solubilizer, a chelator and a nonionic detergent are used in the extraction reagent to replace the need for chaotropes and/or RNase inhihitors.

WO 02/057289 by Invitrogen Corp (2002) discloses methods for isolating RNA from plants in which RNA isolation reagents containing 2-mercaptoethanol, chloroform and isopropyl alcohol are used.

Both methods employ very hazardous reagents.

**Objects of invention:**

The main object of the present invention is ω provide a method for rapid isolation of RNA.

Another object of the present invention is to provide a method for isolation of RNA using two-solution system.

Further, another object of the present invention is to provide a cost effective, less hazardous, two-solution system for rapid isolation of RNA.

Yet another object of the present invention is to provide a two a RNA isolation kit suitable for downstream applications employed in molecular biology includes synthesis of a complementary DNA (cDNA) using RNA as a substrate and performing northern hybridization, polymerase chain reaction, cloning of genes, preparation of probe and construction of a cDNA library.

## Brief description of figures:

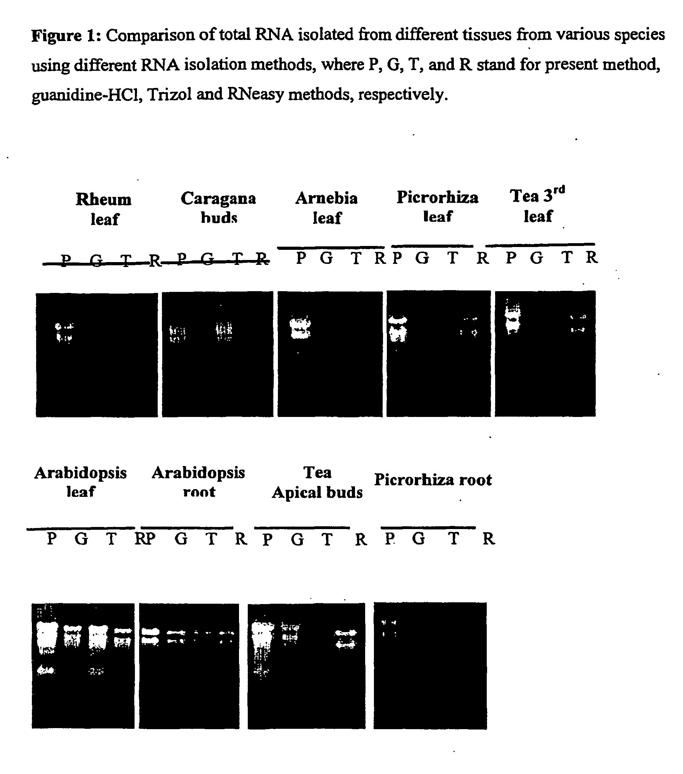

Figure 1 represents comparison of total RNA isolated from different tissues from various species using different RNA isolation methods, where P, G, T, and R stand for present method, guanidine-HCl, Trizol and RNeasy methods, respectively.

Figure 2 represents total RNA isolated from leaf and root of *Arabidopsis thaliana* cv. col-0. A represents RNA isolated from leaf and B represents RNA isolated from root.

Figure 3 represents total RNA isolation from leaf of *Arabidopsis thaliana* cv. col-0 using different quantities of phase separation solution. A, B, C, and D contains 200, 400, 600 and 800 μL of solution II respectively.

Figure 4 represents total RNA isolated from *Solanum tuberosum, Capsicum annum, Rheum, Picrorhiza* leaves and roots. A is RNA from *Solanum* tuber, B is RNA from fruit of *Capsicum,* C is RNA from *Rheum* leaves, D is RNA from *Picrorhiza* leaves and E is RNA from *Picrorhiza* roots.

Figure 5 represents total RNA isolated from vegetative Bud and third leaf of tea. M represents RNA marker; A, RNA isolated from vegetative bud; B, RNA isolated from third leaf

Figure 6 represents RT-PCR based amplification of the RNA of *Arabidopsis thaliana* cv. col-0 using actin primers. M represents 100 bp marker; C represents control reaction without template; A represents amplification with cDNA of *Arabidopsis.*

Figure 7 represents RT-PCR based amplification of the RNA of Rheum using actin primers.

Figure 8 represents RT-PCR based amplification of the RNA of *Arabidopsis thaliana* cv. col-0 using wrky primers. M represents 100 b.p. marker. A represents control reaction with template and B showing amplification of 500 base pairs.

Figure 9 represents RT-PCR based amplification of *Rheum* RNA with *wrky* specific primers. Where M is 100 b.p. marker, A is control reaction without template and B is amplification. product of 700 base pairs.

Figure 10 represents RT-PCR based amplification of the RNA of Vegetative bud and third leaf from tea with DFR specific primers. Where M is 100 base pairs marker, A is amplification in vegetative bud and B is amplification in third leaf.

Figure 11 represents northern hybridization with RNA from tea bud (A) and third leaf (B) using 950 base pairs DFR fragment as probe.

## Summary of invention:

The present invention deals with a cost effective and less hazardous method for rapid isolation of RNA using two solution system. It also provides a rapid RNA isolation kit. It provides RNA from a range of tissues that can be utilized for various downstream applications like RT-PCR, northern hybridizations etc.

## Detailed description of invention:

Accordingly, the present invention provides an RNA isolation system according to claim 1, a method for rapid isolation of RNA according to claim 7 and a rapid RNA isolation kit according to claim 9. RNA RNA

In an embodiment of the present invention, the said tissue is selected from the group consisting of tubers of *Solanum tuberosum,* fruits of *Capsicum annum, Rheum* leaves, leaves and roots of *Picrorhiza kurroa,* leaves and roots of *Arabidopsis thaliana.*

In another embodiment of the present invention, the said solution I comprises of saturated phenol of pH 6.0-6.8, anionic detergent, acetate salt and a chelator wherein the ratio of the individual ingredient is in the range of 10 : 0.003 :

0.02 : 1.0 to 10 : 0.03 : 0.08 : 2.0 respectively.

In further another embodiment of the present invention, saturated phenol used is preferably of a pH less than 7.

In yet another embodiment of the present invention, the anionic detergent used is selected from the group of a dodecyl sulphate salt or N-lauroyl sarcosine.

In still another embodiment of the present invention, the anionic detergent used is in an amount of about 0.3-1% weight/volume most preferably about 0.5-0.6% W/V.

In still another embodiment of the present invention, the acetate salt used is selected from the group of sodium or potassium.

In still another embodiment of the present invention, the acetate salt used is in concentration of 0.2-0.8 M.

In still an embodiment of the present invention, the chelating agent used is selected from the group of disodium and dipotassium of ethylene diamine tetraacetic acid

In still another embodiment of the present invention, the solution II comprising diethylpyrocarbonate in deionised water having a conductivity of 17-18.2 mega-ohms.

In still another embodiment of the present invention, the diethylpyrocarbonate used is in a concentration of about 0.1 % volume /volume.

In still another embodiment of the present invention, the isolated RNA is tested for down-stream applications in molecular biology.

Further, the present invention also provides a rapid RNA isolation kit comprising:

a) solution I;
b) solution II;
c) instructions for using the solutions.

Present invention provides a cost effective, less hazardous, two-solution system for rapid isolation of RNA which includes solution I comprising of buffer saturated phenol (pH less than 7), SDS(0.1-1%), EDTA(10-20mM), sodium acetate(0.3-0.8M), and solution II comprising of DEPC(0.001-0.1%) treated deionized water. Solution system can be used to isolate RNA from diverse tissues. Time required in whole process of isolation of RNA is less than an hour and it provides pure and high quality RNA.

The optimum quantity of solution II was standardized using the leaves of *Arabidopsis thaliana* (a model plant system in plant biology). The developed procedure was employed for a range of plant materials which included tubers of *Solanum tuberosum* (carbohydrate rich), fruits of *Capsicum annum, Rheum* leaves (recalcitrant to guanidine containing solutions), leaves and roots of *Picrorhiza kurroa,* leaves and roots of *Arabidopsis thaliana,* and vegetative buds and third leaf of *Camellia sinensis* (high polyphenol containing tissue). Comparative data of yield of isolated RNA using present protocol and other protocol is given in table 1.

**Table 1**

| Comparative data of yield of isolated RNA | | | | |
|---|---|---|---|---|
| Plant material | Yield μg/10mg tissue | | | |
| | Present invention | Guanidine hydrochloride based method (Logemann et al., 1987) | Trizol (Supplied by Sigma and invitogen, has both phenol and guanidine salt) | RNeasy (Qiagene, GmbH) Phenol free method |
| Rheum | 174 | Nil | Nil | 5.5 |
| Arabidopsis leaf | 153 | 60 | 133.3 | 32.64 |
| Arabidopsis root | 46 | 30 | 15 | 30.1 |
| Arnebia leaf | 152 | Nil | 49.1 | 17.9 |
| Caragana buds (control) | 81 | 25 | 65 | 28.5 |
| Caragana buds (growing under snow in native habitat) | 107 | 28 | 102 | 22.2 |
| Tea buds | 215 | 150 | Nil | 59.5 |

(continued)

| Plant material | Yield µg/10mg tissue | | | |
|---|---|---|---|---|
| | Present invention | Guanidine hydrochloride based method (Logemann et al., 1987) | Trizol (Supplied by Sigma and invitogen, has both phenol and guanidine salt) | RNeasy (Qiagene, GmbH) Phenol free method |
| Tea leaf . | 164 | 30 | Nil | 30 |
| Picrorhiza leaf | 58 | 40 | 15.21 | 18.04 |
| Picrorhiza root | 93 | 54 | 16.96 | 9.84 |

The following examples are given by way of illustration of the present invention and should not be construed to limit the scope of present invention

## EXAMPLE 1

*Preparation of solution for isolation of RNA*

To prepare solution I, phenol was saturated with Tris-(hydroxymethyl)-aminomethane (Tris) buffer to a pH of $6.7 \pm 0.2$. Thereafter, 0.3-1% sodium dodecyl sulphate, 0.2-0.8M sodium acetate and 10-20 mM EDTA was added.
The solution II was prepared separately wherein 0.1% DEPC (final concentration) was added to deionised water having a conductivity of 17-18.2 mega-ohms. Solution was autoclaved after leaving overnight.

## EXAMPLE 2

To isolate total RNA, the following protocol are applied:

*RNA isolation using Guainidine hydrochloride-based procedure:*

(Singh, G., Kumar, S. and Singh, P. 2003. A quick method to isolate RNA from wheat and other carbohydrate-rich seeds. Plant Molecular Biology Reporter 21: 93a-93f)

1. Tissue (1 g) was ground in liquid nitrogen to fine powder. Powder was transferred into a new mortar containing 5 ml of the GH buffer [8 M guanidine hydrochloride, 20 mM ethylene diamine tetraacetic acid (EDTA), 20 mM MES {2-(N-morpholino)ethanesulfonic acid}, beta mercaptoethanol, 200 mM; pH 7.0] and was ground further.
2. Resulting homogenate was transferred to an oak-ridge tube containing equal volume of PCI (Phenol:chloroform: isoamylalcohol).
3. Phases were emulsified by vortexing and separated by centrifugation at 10,000 rpm for 20 min (7°C).
4. Upper aqueous phase was transferred to a fresh oak-ridge tube and extracted with the equal volume of CI.
5. Resulting upper aqueous phase was transferred to a Corex tube and RNA was precipitated by adding 0.2 volume of 1 M acetic acid and 0.7 volume of chilled ethanol.
6. The tubes were kept at -72°C for 3 h.
7. Precipitate was pelleted followed by spin at 10,000 rpm for 10 min at 4°C. Pellet was washed thrice using 5 ml of 3 M sodium acetate (pH 5.2) followed by final washing with 70% chilled ethanol.
8. Pellet was dried and dissolved *in* minimum volume of DEPC-treated ADW.

*RNA isolation using TRIzol Reagent:*

1. Tissue (100 mg) was ground in liquid nitrogen to fine powder.
2. Added 1 ml of TRIzol reagent and further ground to a fine powder using pestle and mortar.
3. Incubated the homogenized samples for 5 min at 15 to 30°C. Added 0.2 ml of chloroform per ml of TRIzol reagent. The tubes were shaken vigorously for 15 seconds and incubated at 15 to 30°C for 2 to 3 min.
4. Centrifuged the samples at 12,000 X g for 15 min at 4°C.
5. Transferred the aqueous phase to a fresh tube.
6. The RNA was precipitated from the aqueous phase by mixing with isopropyl alcohol. Added 0.5 ml of isopropyl alcohol per 1 ml of TRIzol reagent used for initial homogenization.
7. Incubated the samples at 15 to 30°C for 10 min.
8. Centrifuged the samples at 12,000 X g for 10 min at 4°C.
9. Removed the supernatant. Washed the RNA pellet with 75% ethanol and air dry.
10. Dissolved RNA in 30-50 $\mu$l of RNase-free water.

*RNeasy Plant Mini Protocol for RNA isolation: (QLAGEN):*

1. Tissue (100 mg) was ground under liquid nitrogen to a fine powder using a pestle and mortar. Transferred the tissue powder to a 2 ml tube. Do not allow the sample to thaw.
2. Added 450 $\mu$l of buffer RLC (containing guanidine hydrochloride) to maximum of 100 mg tissue powder. Vortexed vigorously. Ensure 10$\mu$l $\beta$-ME is added to 1 ml of buffer RLC before use.
3. Applied lysate to the QIAshredder spin column sitting in a 2 ml collection tube, and centrifuged for 2 min at maximum speed. Transferred the flow-through fraction from QIAshredder to a new tube without disturbing the cell debris pellet in the collection tube.
4. Added 0.5 volumes ethanol to the cleared lysate and mixed well by pipetting.
5. Applied sample, including any precipitate, which may have formed, onto an RNeasy mini spin column sitting in a 2 ml collection tube. Centrifuged for 15 sec at 10,000 rpm.
6. Added 700 $\mu$l of buffer RW onto the RNeasy column, and centrifuged for 15 sec at 10,000 rpm to wash. Discarded the flow-through and collection tube.
7. Transferred RNeasy column into a new 2 ml collection tube. Added 500$\mu$l buffer RPE onto the RNeasy column and centrifuged for 15 sec at 10,000 rpm.
8. Added 500$\mu$l buffer RPE to RNeasy column, and centrifuged for 2 min at maximum speed to dry the RNeasy membrane.
9. Placed the RNeasy spin column in a new 2 ml collection tube, and discarded the old collection tube with filtrate. Centrifuged at full speed for 1 min.
10. Transferred RNeasy column into a new 1.5 ml collection tube, and added 50$\mu$l of RNase-free water directly onto the RNeasy membrane. Centrifuged for 1 min at 10,000 rpm to elute the RNA.

*Isolation of RNA using present protocol:*

a. Leaves of *Arabidopsis thaliana* (100 mg) were ground in liquid nitrogen using mortar and pestle to make fine powder.
b. Added 2 ml of solution I.
c. Homogenized to make a fine powder in pestle.
d. Added 800$\mu$l solution II
e. Transferred sample into 2.0 ml eppendorf tubes.
f. Added 200$\mu$l of chloroform.
g. Vortexed and left at room temperature for 10 minutes.
h. Centrifuged for 10 minutes at room temperature
i. Transferred upper aqueous phase into a new 2.0 ml eppendorf tube.
j. Added 0.6 Volumes of isopropanol
k. Vortexed and left at room temperature for 10 minutes.
l. Centrifuged for five minutes at 4°C.
m. Resulting RNA pellet was washed with 70% ethanol, air-dried and dissolved in appropriate amount of DEPC treated water.

RNA was quantified by scanning absorbance between 220 to 320 nm; the purity was determined by calculating the ratio of absorbance measured at 260 and 280 nm. A value >1.8 at 260/280 nm was considered ideal for the purpose of present investigation. The formula used to calculate RNA concentration and yield was as follows:

$$\text{Concentration of RNA } (\mu g/ml) = A_{260} \text{ (Absorbance at 260 nm) X 40 X dilution factor}$$

$$\text{Total yield } (\mu g) = \text{concentration X volume of stock RNA sample.}$$

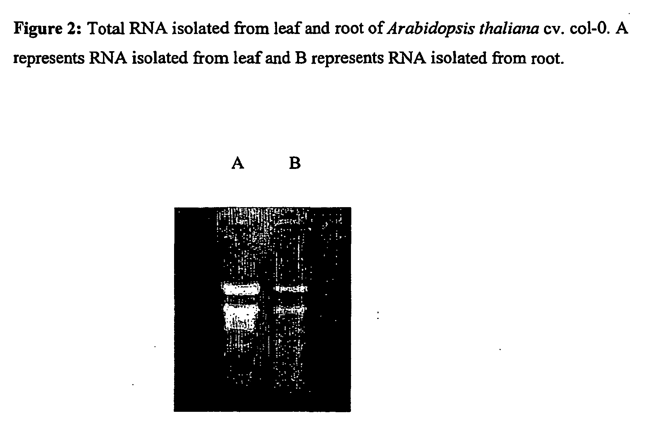

The quality of RNA isolated was assayed on 1.2% agarose gel containing formaldehyde. To check the integrity of RNA, 5-6 $\mu$g of RNA in 4.5 $\mu$l of DEPC treated autoclaved water was diluted with 15.5 $\mu$l of M1 solution (2 $\mu$l of X MOPS buffer, 3.5 $\mu$l of formaldehyde and 10 $\mu$l of formamide [5X MOPS buffer: 300mM sodium acetate, 10mM MOPS (3-{N-morpholino propane sulphonic acid}, 0.5 mM ethylene diamine tetra-acetic acid (EDTA)] and incubated for 15 minutes at 65 degree C. RNA was loaded onto 1.2% formaldehyde agarose-gel after adding 2µl of formaldehyde- gel loading buffer [50% glycerol, 1mM EDTA (pH 8.0), 0.25% bromophenol blue, 0.25% xylene cyanol FF] and electrophoresed at 72 volts in 1X MOPS buffer (60mM sodium acetate, 2mM MOPS and 0.1 mM EDTA),as described by Sambrook, J., Fritsch, E.F., Maniatis, T., (1989) ( in Molecular Cloning: A Laboratory Manual, Cold Spring harbor Laboratory Press, Plainview, NY). Using 100 mg of leaves 153 µg RNA was obtained (Table 2). Figure 2 shows that the isolated RNA was intact as it revealed intact bands.

Table 2

| Yield of RNA from different tissues | |
| --- | --- |
| Plant material | Yield of RNA (µg/100 mg tissue) |
| Arabidopsis Leaves | 153 |
| Arabidopsis Roots | 46 |
| Picrorhiza Leaves | 58 |
| Picrorhiza Roots | 93 |
| Rheum Leaves | 174 |
| Tea 3rd Leaf | 164 |
| Tea Buds | 215 |
| Caragana buds (Control) | 81 |
| Caragana buds (Under stress) | 107 |
| Arnebia Leaf | 152 |
| Arnebia Root | 136 |
| Capsicum Fruit | 49 |
| Solanum Tuber | 29 |

Comparative data of yield of isolated RNA using present protocol and other protocol mentioned in example 2 is given in table 1.

## EXAMPLE 3

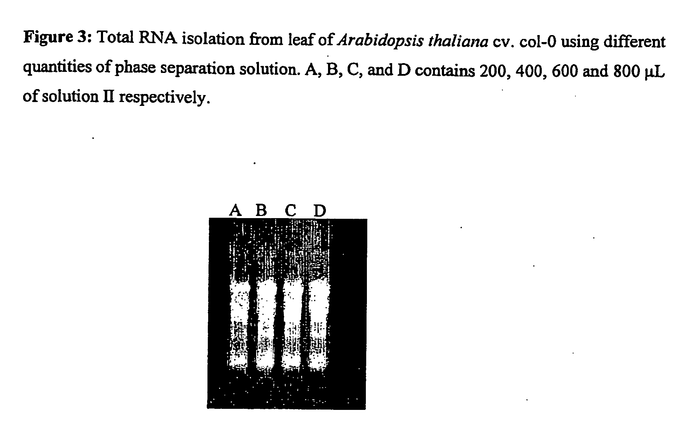

The amount of solution II added to the homogenate was varied to study its effect on RNA quantity and quality. *Arabidopsis thaliana* leaves were used as starting material and the protocol was followed essentially as described except that 200µl, 400µl, 600µl and 800µl of solution II was added to the homogenate. It is evident from Table 2 and Figure 3 that 800 µl of solution II yields the best results.

Table3

| Effect of different volume of solution II on RNA yield. | |
| --- | --- |
| Volume of Solution II | RNA Yield (µg/100mg tissue) |
| 200 µl | 39.8 |
| 400 µl | 112.6 |
| 600 µl | 116 |
| 800 µl | 134 |

## EXAMPLE 4

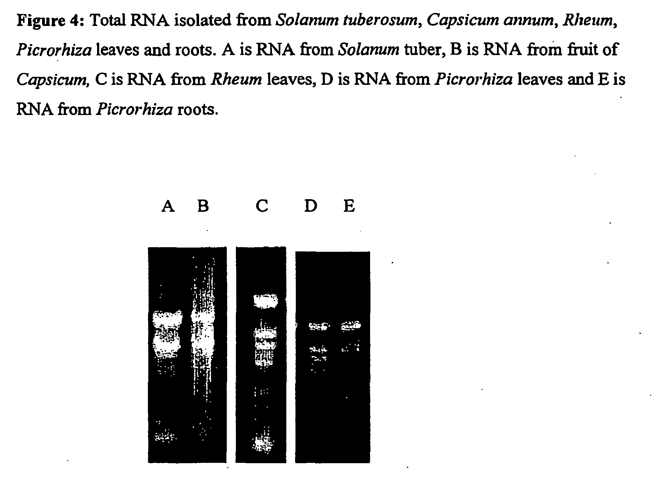

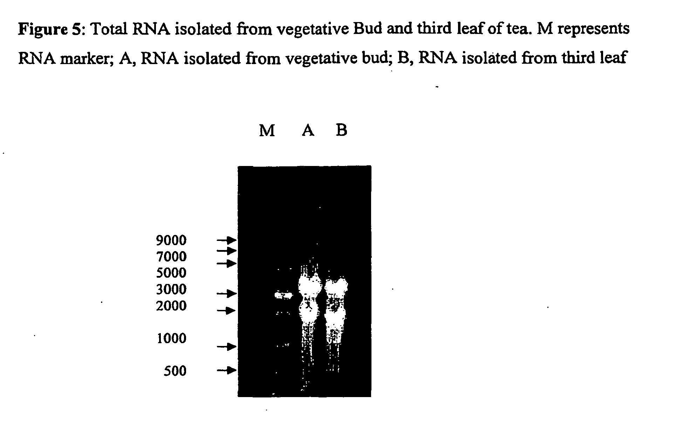

Developed solution was used to isolate RNA from diverse plant species. The samples used were *Solanum tuberosum* tubers (carbohydrate rich) (Figure 4-A), *Capsicum annum* fruits (Figure 4-B), *Rheum* leaves (recalcitrant to guanidine containing solutions) (Figure 3-C), *Picrorhiza* leaves (Figure 4-D) and roots (Figure 4-E), *Arabidopsis* leaves and roots (Figure 2), and *Camellia sinensis* (Tea) vegetative buds and third leaf (rich in ployphenols) (Figure 5). The procedures used for isolation, quantity and quality assessment of RNA are the same as described in example 2. Depending upon the tissue used, the yield can be as high as 215 $\mu$g/100 mg of tissue. Table 1 describes the yield obtained from different tissues.

## EXAMPLE 5

To assess the suitability of RNA for downstream applications, a reverse transcription (RT) polymerase chain reaction (PCR) was performed using the RNA from *Arabidopsis* roots and shoots, and *Rheum* leaves. Total RNA (2 $\mu$g) was digested with DNase I (Cat. No. 18068-015 Invitrogen, U.S.A.), reverse-transcribed with superscript II reverse transcriptase (Cat No. 18064-022, Invitrogen, U.S.A.) to synthesize cDNA using oligo dT primer (Cat. No. 18418-012, Invitrogen, U.S.A.). In both the above reactions, manufacturer's instructions were followed. For PCR amplification, oligonucleotide primers specific to beta actin were used to amplify this DNA during 35 cycles, where a cycle was defined as 94 degree C for30 seconds, 52 degree C for 1 minute, and 72 degree for 1 minute. The composition of reaction mixture was as follows:

| Reagents | Quantity in $\mu$l |
| --- | --- |
| 10X PCR buffer (20 mM Tris-HCl, pH 8.4, 50 mM KCL, 1.5 mM MgCl$_2$) | 2.5 |
| Deoxy nucleotide triphosphate(dNTPs) 10Mm | 0.5 |
| Forward primer (2$\mu$M) | 2.5 |
| Reverse primer(2$\mu$M) | 2.5 |
| cDNA (as in example 5) | 1 |
| Taq DNA polymerase (5U/$\mu$L) | 0.2 |
| Sterile distilled water | 15.8 |
| Total | 25 |

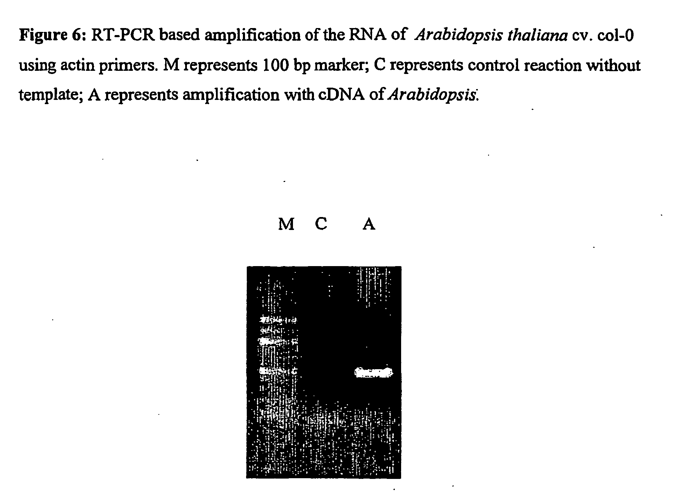

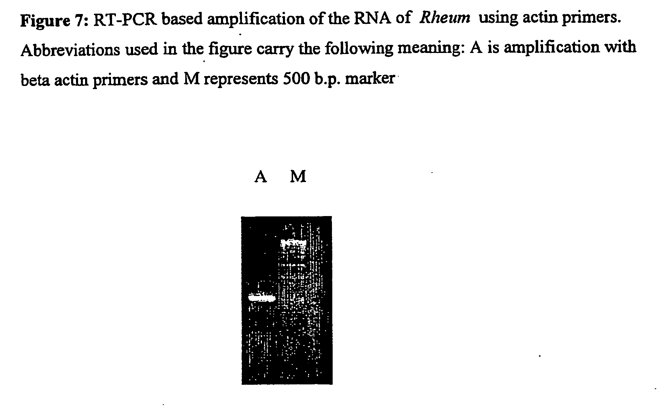

The amplified DNA was electrophoresed through 1.5% agarose gel. A band of 500 base pairs was amplified amplification from cDNA of *Arabidopsis* (Figure 6) and *Rheum* (Figure 7).

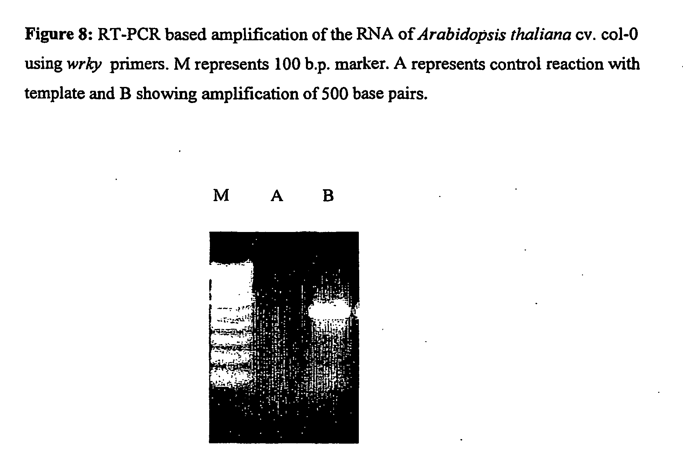

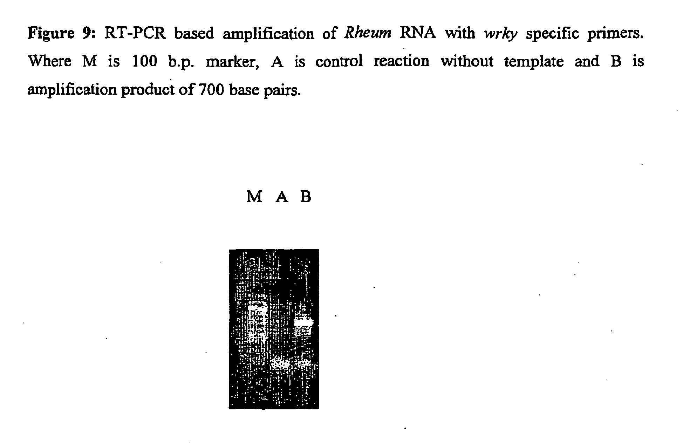

Using cDNA synthesized from *Arabidopsis* and *Rheum* RNA another PCR was carried out with oligonucleotide primers specific for transcription factor, *wrky*. The cycling parameters for wrky primers to amplify DNA for 35 cycles were 94 degree C for 30 seconds, 48 degree C for 45 seconds and 72 degree for 1 minute. The amplified products were visualized on 1.5% agarose gel. The amplification products of 500 base pairs (Figure No. 8) and 700 base pairs (Figure No. 9) were obtained in *Arabidopsis* and *Rheum,* respectively. Sequencing confirmed them to be the *wrky* fragments.

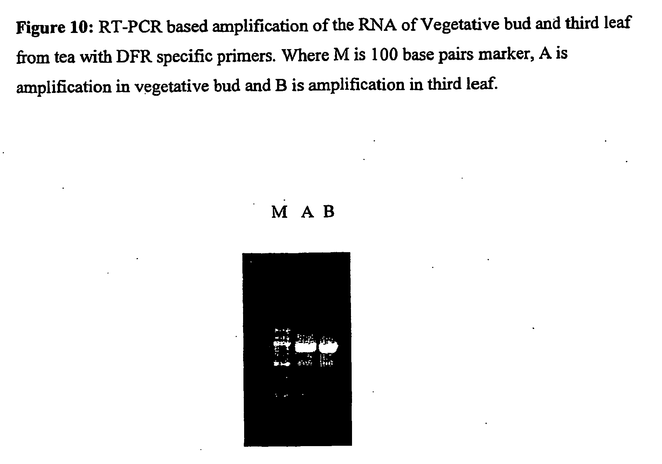

Complementary DNA was also synthesized using RNA isolated from tea buds and third leaf essentially as described elsewhere. For PCR amplification, oligonucleotide primers specific for dihydroflavonol-4- reductase (DFR) were used for amplification during 35 cycles, where cycling parameters were defined as 94 degree C for 30 sec., 52 degree C for 40 sec., and 72 degree for 2 minutes. The amplified DNA was electrophoresed through 1.5% agarose gel at 80 volts for 2 hours. As evident from Figure 10, a band of 950 base-pair was amplified and further sequencing confirmed it to be the fragment of DFR.

Thus, the experiments explicitly showed that isolated RNA is suitable for RT-PCR analysis, a down-stream application.

## EXAMPLE 6

To further assess the suitability of RNA for down-stream applications, northern blotting was performed using RNA from tea bud and third leaf using the 950 base pairs amplification product as obtained in example 5 as a probe. After visualizing the amplified products on 1.5% agarose gel. The band was excised from the gel and DNA was eluted using QIAEX II Gel Extraction Kit from M/s Qiagen, Germany, following the manufacturer's instructions.

Purified fragment was radiolabelled with $\alpha$- [$^{32}$P] dATP (4000 Ci/mmole) using HotPrime Kit (M/s GenHunter Corporation, Nashville, U.S.A.). Unincorporated radionucleotides were removed using QIAquick Nucleotide Removal Kit (QIAGEN, Germany). Radiolabelled probe was used for hybridization experiments.

For blotting, 15 $\mu$g of RNA was elecrophoresed on 1.0% formaldehyde agarose gel essentially as described in example 2. Following electrophoresis, the gel was washed twice with DEPC treated autoclaved water for 15 minutes each with shaking. Gel was then washed twice with 10 X SSPE (10X SSPE: 1.5 M sodium chloride, 115 mM $NaH_2PO_4$, 10mM EDTA) for 20 minutes each, with shaking. Nylon membrane was wetted in DEPC water and then soaked in 10 X SSPE for 5 minutes with gentle shaking. RNA was then vacuum-blotted (Using pressure of 40 mbar) onto nylon membrane using DEPC treated 10X SSPE as transfer medium. Transfer was carried out for 4 hours. Pressure was increased to 70 mbar for 15 minutes before removing the gel from the vacuum blotter. The location of RNA marker was marked on the nylon surface under a UV light source. Membrane was dried and baked at 80 degree C for two hours. After a brief rinse in 5X SSPE membrane was soaked into prehybridization solution (50% formamide, 0.75 M NaCl, 50mM sodium phosphate, pH 7.4, 0.5 mM EDTA, 0.1 % Ficoll-400, 0.1% bovine serum albumin, 0.1% polyvinylpyrollidone, 0.1% SDS solution and 150 μg of freshly boiled salmon sperm DNA) for five hours.

Radiolabelled probe was denatured by boiling for 10 minutes followed by addition to the prehybridization solution. Hybridization was carried out for 16 hours. Solution was removed and the membrane was washed twice with 1X SSC (20X SSC; 3M sodium acetate and 0.3M sodium citrate dihydrate, pH 7.0) containing 0.1% SDS at room temperature for 15 minutes each. Final washing was done at 60 degree C (Using prewarmed 0.25X SSC containing 0.1 % SDS for 15 minutes. Membrane was wrapped in saran wrap and exposed to X-ray film for 48 hours.

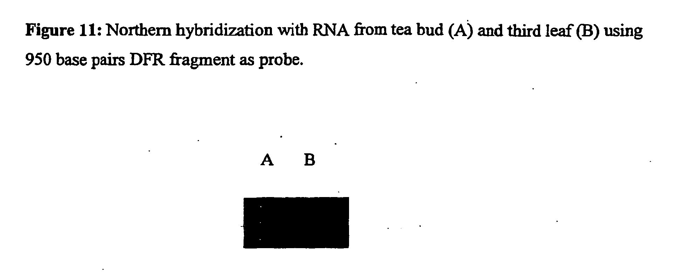

Figure 11 shows that probe did give signal. This result further confirmed that isolated RNA is suitable for down stream applications.

### Advantages:

The main advantages of the present invention are:

1. Present invention provides good quality of RNA.
2. Good quality and quantity of RNA can be isolated using present protocol.
3. This method can be applied for various tissue system.
4. Present invention provides cost effective and rapid method for RNA isolation.
5. The invention is suitable for tissues recalcitrant to guanidine salts

## Claims

1. An RNA isolation system consisting of solution I and solution II, wherein the said solution I consists of saturated phenol of pH 6.0-6.8, anionic detergent, acetate salt and a chelator wherein the ratio of the individual ingredient is in the range of 10: 0.003: 0.02: 1.0 to 10: 0.03: 0.08: 2.0 respectively and solution II consists of diethylpyrocarbonate in deionised water having a conductivity of 17-18.2 mega-ohms.

2. A solution as claimed in claim 1, wherein the phenol is saturated with the Tris-(hydroxymethyl)-aminomethane (Tris) buffer.

3. A solution as claimed in claim 1, wherein the anionic detergent used is selected from the group of a dodecyl sulphate salt or N-lauroyl sarcosine.

4. A solution as claimed in claim 1, wherein the acetate salt used is selected from the group of sodium or potassium.

5. A solution as claimed in claim 1, wherein the chelating agent used is selected from the group of disodium and dipotassium of ethylene diamine tetraacetic acid.

6. A solution as claimed in claim 1, wherein the diethylpyrocarbonate used is in a concentration of about 0.1% volume /volume.

7. A method for rapid isolation of RNA, wherein the said method comprises the steps of:

a) grinding the plant tissue in liquid nitrogen to make it fine powder;
b) adding solution I consisting of saturated phenol of pH 6.0-6.8, anionic detergent, acetate salt and a chelator wherein the ratio of the individual ingredient is in the range of 10: 0.003: 0.02: 1.0 to 10: 0.03: 0.08:2.0 respectively in the powdered sample obtained from step (a) in the ratio ranging from 1 :10 to 1 :50 followed by homogenizing to make it a fine powder;
c) adding solution II consisting of diethylpyrocarbonate in deionised water having a conductivity of 17-18.2 mega- ohms in powdered sample obtained from step (b); wherein the concentration of solution I to solution II used is in the ratio ranging from 5:3 to 5:2;

d) adding chloroform to the above said solution obtained from step (c) followed by vortexing and kept it at room temperature for 10 min to separate the layer;

e) transferring upper layer obtained from step (d) into fresh tube;

f) adding isopropanol into above layer obtained from step (e) in the ratio of 5:3 to 5:4 followed by vortexing and kept it at room temperature for 10 min;

g) centrifuging the solution obtained from step (f) for 5 to 10 min at about 4°C to get the desired RNA pellet;

h) washing the resultant RNA pellets obtained from step (g) with 70% ethanol followed by air drying;

i) dissolving the washed pellet obtained from step (h) in appropriate amount of DEPC treated water.

8. A method as claimed in claim 7, wherein the said tissue is selected from the group consisting of tubers of *Solanum tuberosum,* fruits of *Capsicum annum, Rheum* leaves, leaves and roots of *Picrorhiza kurroa,* leaves and roots of *Arabidopsis thaliana.*

9. Rapid RNA isolation kit comprising:

a) solution I consisting of saturated phenol of pH 6.0-6.8, anionic detergent, acetate salt and a chelator wherein the ratio of the individual ingredient is in the range of 10: 0.003: 0.02: 1.0 to 10: 0.03: 0.08: 2.0 respectively;

b) solution II consisting of diethylpyrocarbonate in deionised water having a conductivity of 17-18.2 mega- ohms;

c) instructions for using the solutions.

**Patentansprüche**

1. RNA-Isolationssystem bestehend aus einer Lösung I und einer Lösung II, wobei die Lösung I aus gesättigtem Phenol mit einem pH-Wert von 6,0 - 6,8, einem anionischen Detergens, einem Acetatsalz und einem Chelator besteht, wobei das Verhältnis der einzelnen Bestandteile im Bereich von jeweils 10:0,003:0,02:1,0 bis 10:0,03:0,08:2,0 liegt, und die Lösung II aus Diethylpyrocarbonat in deionisiertem Wasser besteht, das eine Leitfähigkeit von 17 - 18,2 Megaohm hat.

2. Lösung nach Anspruch 1, wobei das Phenol mit Tris-(hydroxymethyl)-aminomethan-(Tris)-Puffer gesättigt ist.

3. Lösung nach Anspruch 1, wobei das verwendete anionische Detergens ausgewählt ist aus der Gruppe bestehend aus einem Dodecylsulfatsalz oder N-Lauroylsarcosin.

4. Lösung nach Anspruch 1, wobei das verwendete Acetatsalz ausgewählt ist aus der Gruppe bestehend aus Natrium- oder Kaliumsalz.

5. Lösung nach Anspruch 1, wobei der Chelatbildner ausgewählt ist aus der Gruppe bestehend aus Dinatrium- und Dikalium-Ethylendiamintetraessigsäure.

6. Lösung nach Anspruch 1, wobei das verwendete Diethylpyrocarbonat in einer Konzentration von etwa 0,1 % Vol./Vol. vorliegt.

7. Verfahren zur schnellen Isolation von RNA, wobei das Verfahren die folgenden Schritte umfasst:

a) Mahlen des Pflanzengewebes in flüssigem Stickstoff, um ein feines Pulver daraus zu machen;

b) Hinzufügen der Lösung I, die aus gesättigtem Phenol mit einem pH-Wert von 6,0 - 6,8, einem anionischen Detergens, einem Acetatsalz und einem Chelator besteht, wobei das Verhältnis der einzelnen Bestandteile im Bereich von jeweils 10 : 0,003 : 0,02 : 1,0 bis 10 : 0,03 : 0,08 : 2,0 liegt, zu der pulverisierten Probe aus Schritt (a) im Verhältnis im Bereich von 1 :10 bis 1 :50, mit anschließender Homogenisierung, um ein feines Pulver daraus zu machen;

c) Hinzufügen der Lösung II, die aus Diethylpyrocarbonat in deionisiertem Wasser besteht, das eine Leitfähigkeit von 17 - 18,2 Megaohm hat, zu der pulverisierten Probe, die in Schritt b) erhalten wurde, wobei das Verhältnis der Konzentration der verwendeten Lösung I zur Konzentration der verwendeten Lösung II im Bereich von 5 : 3 bis 5 : 2 ist;

d) Hinzufügen von Chloroform zu der vorstehend genannten in Schritt c) erhaltenen Lösung mit anschließendem Verwirbeln und Aufbewahren bei Raumtemperatur für 10 Minuten, um die Schichten zu trennen;

e) Überführen der oberen in Schritt (d) erhaltenen Schicht in ein neues Röhrchen;

f) Hinzufügen von Isopropanol zu der vorstehend genannten in Schritt e) erhaltenen Schicht im Verhältnis von 5 : 3 bis 5 : 4 mit anschließendem Verwirbeln und Aufbewahren bei Raumtemperatur für 10 Minuten;

g) Zentrifugieren der in Schritt f) erhaltenen Lösung für 5 bis 10 Minuten bei etwa 4°C, um das gewünschte RNA-Pellet zu erhalten;

h) Waschen der so in Schritt g) erhaltenen RNA-Pellets mit 70% Ethanol mit anschließender Lufttrocknung;

i) Auflösen des in Schritt h) erhaltenen gewaschenen Pellets in einer geeigneten Menge von DEPC-behandeltem Wasser.

8. Verfahren nach Anspruch 7, wobei das Gewebe ausgewählt ist aus der Gruppe bestehend aus Knollen von *Solanum tuberosum,* Früchten von *Capsicum annum,* Rheum-Blättern, Blättern und Wurzeln von *Picrorhiza kurroa,* Blättern und Wurzeln von *Arabidopsis thaliana.*

9. Kit für die schnelle RNA-Isolation, umfassend:

a) Lösung I bestehend aus gesättigtem Phenol mit einem pH-Wert von 6,0 - 6,8, einem anionischen Detergens, einem Acetatsalz und einem Chelator, wobei das Verhältnis der einzelnen Bestandteile im Bereich von jeweils 10 : 0,003 : 0,02 : 1,0 bis 10 : 0,03 : 0,08 : 2,0 liegt;

b) Lösung II bestehend aus Diethylpyrocarbonat in deionisiertem Wasser mit einer Leitfähigkeit von 17 - 18,2 Megaohm;

c) Instruktionen für den Gebrauch der Lösungen.

**Revendications**

1. Système d'isolement de l'ARN consistant en une solution I et une solution II, dans lequel ladite solution I consiste en du phénol saturé à un pH de 6,0-6,8, un détergent anionique, un sel d'acétate et un agent chélatant, où le rapport de l'ingrédient individuel est dans la plage de 10:0,003:0,02:1,0 à 10:0,03:0,08:2,0 respectivement, et la solution II consiste en du diéthylpyrocarbonate dans de l'eau désionisée ayant une conductivité de 17-18,2 méga-ohms.

2. Solution selon la revendication 1, dans laquelle le phénol est saturé avec le tampon Tris-(hydroxyméthyl)-aminométhane (Tris).

3. Solution selon la revendication 1, dans laquelle le détergent anionique utilisé est sélectionné dans le groupe contenant un sel de dodécylsulfate ou la N-lauroylsarcosine.

4. Solution selon la revendication 1, dans laquelle le sel d'acétate utilisé est sélectionné dans le groupe contenant le sodium ou le potassium.

5. Solution selon la revendication 1, dans laquelle l'agent chélatant utilisé est sélectionné dans le groupe contenant le disodium et le dipotassium d'acide éthylène diamine tétraacétique.

6. Solution selon la revendication 1, dans laquelle le diéthylpyrocarbonate utilisé est présent en une concentration d'environ 0,1 % en volume/volume.

7. Procédé d'isolement rapide de l'ARN, où ledit procédé comprend les étapes consistant à :

a) broyer le tissu végétal dans de l'azote liquide pour le transformer en poudre fine ;

b) ajouter la solution I consistant en du phénol saturé à un pH de 6,0-6,8, un détergent anionique, un sel d'acétate et un agent chélatant, où le rapport de l'ingrédient individuel est dans la plage de 10:0,003:0,02:1,0 à 10:0,03:0,08:2,0 respectivement, dans l'échantillon en poudre obtenu à l'étape (a) selon un rapport compris entre 1:10 et 1:50 suivi d'une homogénéisation afin de le transformer en poudre fine ;

c) ajouter la solution II consistant en du diéthylpyrocarbonate dans de l'eau désionisée ayant une conductivité de 17-18,2 méga-ohms dans l'échantillon en poudre obtenu à l'étape (b) ; où la concentration de la solution I sur la solution II utilisée est selon un rapport compris entre 5:3 et 5:2 ;

d) ajouter du chloroforme à ladite solution ci-dessus obtenue à l'étape (c) suivi d'une agitation au vortex et la maintenir à température ambiante pendant 10 minutes pour séparer la couche;

e) transférer la couche supérieure obtenue à l'étape (d) dans un tube neuf ;

f) ajouter de l'isopropanol dans la couche ci-dessus obtenue à l'étape (e) selon un rapport de 5:3 à 5:4 suivi d'une agitation au vortex et la maintenir à température ambiante pendant 10 minutes ;

g) centrifuger la solution obtenue à l'étape (f) pendant 5 à 10 minutes à environ 4 °C afin d'obtenir le culot d'ARN souhaité ;

h) laver les culots d'ARN résultants obtenus à l'étape (g) avec de l'éthanol à 70 % suivi d'un séchage à l'air ;

i) dissoudre le culot lavé obtenu à l'étape (h) dans une quantité appropriée d'eau traitée par du DEPC.

8. Procédé selon la revendication 7, dans lequel ledit tissu est sélectionné dans le groupe consistant en des tubercules de *Solanum tuberosum,* des fruits de *Capsicum annum,* des feuilles de *Rheum,* des feuilles et des racines de *Picrorhiza kurroa,* des feuilles et des racines *d'Arabidopsis thaliana.*

9. Kit d'isolement rapide de l'ARN comprenant :

a) une solution I consistant en du phénol saturé à un pH de 6,0-6,8, un détergent anionique, un sel d'acétate et un agent chélatant, où le rapport de l'ingrédient individuel est dans la plage de 10:0,003:0,02:1,0 à 10:0,03: 0,08:2,0 respectivement ;

b) une solution II consistant en du diéthylpyrocarbonate dans de l'eau désionisée ayant une conductivité de 17-18,2 méga-ohms ;

c) des instructions pour utiliser les solutions.

Figure 1: Comparison of total RNA isolated from different tissues from various species using different RNA isolation methods, where P, G, T, and R stand for present method, guanidine-HCl, Trizol and RNeasy methods, respectively.

**Figure 2:** Total RNA isolated from leaf and root of *Arabidopsis thaliana* cv. col-0. A represents RNA isolated from leaf and B represents RNA isolated from root.

A    B

**Figure 3:** Total RNA isolation from leaf of *Arabidopsis thaliana* cv. col-0 using different quantities of phase separation solution. A, B, C, and D contains 200, 400, 600 and 800 μL of solution II respectively.

Figure 4: Total RNA isolated from *Solanum tuberosum*, *Capsicum annum*, *Rheum*, *Picrorhiza* leaves and roots. A is RNA from *Solanum* tuber, B is RNA from fruit of *Capsicum*, C is RNA from *Rheum* leaves, D is RNA from *Picrorhiza* leaves and E is RNA from *Picrorhiza* roots.

**Figure 5**: Total RNA isolated from vegetative Bud and third leaf of tea. M represents RNA marker; A, RNA isolated from vegetative bud; B, RNA isolated from third leaf

**Figure 6:** RT-PCR based amplification of the RNA of *Arabidopsis thaliana* cv. col-0 using actin primers. M represents 100 bp marker; C represents control reaction without template; A represents amplification with cDNA of *Arabidopsis*.

M  C  A

**Figure 7:** RT-PCR based amplification of the RNA of *Rheum* using actin primers. Abbreviations used in the figure carry the following meaning: A is amplification with beta actin primers and M represents 500 b.p. marker

A    M

**Figure 8:** RT-PCR based amplification of the RNA of *Arabidopsis thaliana* cv. col-0 using *wrky* primers. M represents 100 b.p. marker. A represents control reaction with template and B showing amplification of 500 base pairs.

**Figure 9:** RT-PCR based amplification of *Rheum* RNA with *wrky* specific primers. Where M is 100 b.p. marker, A is control reaction without template and B is amplification product of 700 base pairs.

M A B

Figure 10: RT-PCR based amplification of the RNA of Vegetative bud and third leaf from tea with DFR specific primers. Where M is 100 base pairs marker, A is amplification in vegetative bud and B is amplification in third leaf.

M  A  B

**Figure 11:** Northern hybridization with RNA from tea bud (A) and third leaf (B) using 950 base pairs DFR fragment as probe.

A    B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4843155 A
- US 5945515 A, Chomczynski, P.
- US 5777099 A, Mehra, M.
- US 5010183 A, Macfarlane, D. E.
- US 5300635 A, Macfarlane, D. E.
- US 20040019196 A, Bair, R J Jr., Heath, EM., Meehan, H, Paulsen, K E, Wages, J M. Jr.
- US 5973137 A, Heath, E M.
- WO 9845311 A
- WO 02057289 A

### Non-patent literature cited in the description

- Current Protocols in Molecular Biology. John Wiley and Sons, Inc. USA, 1994
- COX, R.A. Methods in Enzymology. Academic Press, 1968, vol. 12 B, 120-129
- R.C. BUGOS ; V.L. CHIANG ; X-H. ZHANG ; E.R. CAMPBELL ; G.K. PODILA ; W.H. CAMPBELL. RNA isolation from plant tissues recalcitrant to extraction in guanidine. *Biotechniques,* 1995, vol. 19, 734-737
- CHIRGWIN, J.M. ; PRZYBYLA, A.E. ; MACDONALD, R.J. ; RUTTER, W.J. *Biochemistry,* 1979, vol. 18, 5294-5299
- WALLACE, D.M. *Methods in Enzymology,* 1987, vol. 152, 33-41
- CHOMCZYNSKI, P. ; SACCHI, N. *Anal. Biochem.,* 1987, vol. 162, 156-159
- SIEBERT, P.D. ; CHENCHIK, A. *Nucl. Acid Res.,* 1993, vol. 21 (8), 2019-2020
- FERAMISCO, J.R. ; HELFMAN, D.M. ; SMART, J.E. ; BURRIDGE, K. ; THOMAS, G.P. Molecular Cloning. Cold Spring Harbor Laboratory, 1982, 194-195
- SINGH, G. ; KUMAR, S. ; SINGH, P. A quick method to isolate RNA from wheat and other carbohydrate-rich seeds. *Plant Molecular Biology Reporter,* 2003, vol. 21, 93a-93f
- SAMBROOK, J. ; FRITSCH, E.F. ; MANIATIS, T. Molecular Cloning: A Laboratory Manual. Cold Spring harbor Laboratory Press, 1989